# EUROPEAN PATENT SPECIFICATION

(11) **EP 1 724 257 B1**
(45) Date of publication and mention of the grant of the patent: **13.11.2013**
(21) Application number: 05719421.9
(22) Date of filing: 23.02.2005
(51) Int. Cl.: C07D 209/08, A61K 31/4045, A61P 9/00, A61P 13/10, A61P 19/00, A61P 25/02, A61P 25/16, A61P 25/28, A61P 43/00

(54) **MEDICINAL COMPOSITION FOR PREVENTION OR TREATMENT OF OVERACTIVE BLADDER ACCOMPANYING NERVOUS DISORDER**
MEDIZINISCHE ZUSAMMENSETZUNG ZUR PRÄVENTION ODER BEHANDLUNG VON MIT HYPERAKTIVER BLASE ASSOZIIERTEN NERVÖSEN ERKRANKUNGEN
COMPOSITION THERAPEUTIQUE DESTINEE A PREVENIR OU TRAITER UNE VESSIE HYPERACTIVE QUI ACCOMPAGNE UN TROUBLE NERVEUX

(30) Priority: 05.03.2004 JP 2004061476
(43) Date of publication of application: 22.11.2006
(73) Proprietor: Kissei Pharmaceutical Co., Ltd., Matsumoto-shi Nagano 399-8710 (JP)
(72) Inventor: YAMAZAKI, Yoshinobu Kissei Pharmaceutical Co., Ltd, -machi, Minamiazumi-gun, Nagano 3998304 (JP); IMAMURA, Takahiro, Kissei Pharmaceutical Co., Ltd., -machi, Minamiazumi-gun, Nagano 3998304 (JP)
(74) Representative: Hayes, Adrian Chetwynd
(86) International application number: PCT/JP2005/002913
(87) International publication number: WO 2005/085195

(56) References cited:
- WO-A1-99/15202
- WO-A1-99/48530
- WO-A1-2004/022538
- WO-A1-2004/054574
- JP-A- 2000 247 998
- JP-A- 2001 288 115
- US-A1- 2002 143 007
- YAMADA S. ET AL: 'In vivo receptor binding of novel <SYM97>1-adrenoceptor antagonist for treatment of benign prostatic hyperplasia.' LIFE SCIENCE. vol. 62, no. 17-18, 1998, pages 1585 - 1589, XP002992441
- OKURA T. ET AL: 'Selective and sustained occupancyof prostatic <SYM97>1Aadrenoceptor antagonist by oral administration of KMD-3213 and its plasma concentration in rats.' JOURNAL OF PHARMACY AND PHARMACOLOGY. vol. 54, no. 7, 2002, pages 975 - 998, XP002992444
- AKIYAMA K. ET AL: 'Effects of KMD-3213, a uroselective <SYM97>1-adrenoceptor antagonist of the tilt-induced blood pressure response in Pharmacology.' JAPANESE JOURNAL OF PHARMACOLOGY. vol. 90, no. 2, 2002, pages 131 - 137, XP002992442
- OHASHI T. ET AL: 'Hainyo Shogai to Nyo Shikkin (Ge).' HEKKAN HONDANREN. no. 591, 1998, pages 57 - 60, XP002992445
- YOSHIDA M. ET AL: 'Kabu Nyoro Kino Kenkyu no Saikin no Shinpo: Kiso Kenkyu o Rinsho Oyo ni Tsugu 3 Kabu Nyoro Heikatsukin Kino ni Kakawaru Shinkei Dentatsu Busshitsu no Yakurigakuteki Bunseki.' FOLIA PHARMACOLOGICA JAPONICA. vol. 121, no. 5, 2003, pages 307 - 316, XP002992443
- ISHIDURA O. ET AL: 'Koreisha no Zenritsusen Shikkan Zenritsusen Shikkan to Kakatsudo Boko.' GERIATR.MED. vol. 41, no. 8, 2003, pages 1149 - 1153, XP002992447
- ISHIZUKA O. ET AL: 'Micturition in concious rats with and without bladder outlet obstruction: role of spinal alpha-adrenoceptors.' BR.J. PHARMACOL. vol. 117, no. 5, 1996, pages 962 - 966, XP002992446
- R. A. APPELL: "Overactive Bladder in Special Patient Populations", REVIEWS IN UROLOGY, vol. 5, no. 8, 2003, pages S37-S41,

## Description

### Technical Field

The present invention relates to pharmaceutical compositions which are useful for the prevention or treatment of overactive bladder accompanied with neurogenic disorders.

More particularly, the present invention relates to a pharmaceutical composition for the prevention or treatment of urinary urgency or frequency in overactive bladder accompanied with neurogenic disorders, which comprises as an active ingredient (-)-1-(3-hydroxypropyl)-5-((2R)-2-{[2-({2-[(2,2,2-trifluoroethyl)-oxy]phenyl}oxy)ethyl]amino}propyl)-2,3-dihydro-1H-indol-7-carboxamide or a pharmaceutically acceptable salt thereof.

### Background Art

Overactive bladder (OAB) is defined as a disease based on symptoms of urgency, usually with frequency and with or without urge incontinence. The definition of OAB is proposed in the new standardization of terminology reported by the International Continence Society (ICS) (see non-Patent Reference 1). From the epidemiologic survey conducted as a research project by Japanese Neurogenic Bladder Society, it is estimated that there are more than 8 million patients with OAB now (see non-Patent Reference 2).

OAB mostly develops accompanied with neurogenic disorder, lower urinary tract obstruction and others in addition to the ideopatic OAB without defined causes, and various therapeutic methods are performed depending on the pathogenesis (see non-Patent Reference 3). As for the OAB accompanied with neurogenic disorders, neurogenic disorder induced by origin disorder such as brain blood disorder, Parkinson's disease, spinal cord injury or the like causes the unusual micturition control, sensory disorder or the like, and that causes OAB. Thus, nevertheless many therapeutic methods are attempted to improve the urgency, frequency or the like of OAB, enough therapeutic effects are not necessarily obtained. Therefore, the establishment of the new therapeutic method suitable for each origin disorder or disease aspect has been desired.

Now, as the therapeutic methods for OAB accompanied with neurogenic disorder, combination of behavioural modification to establish the normal voiding pattern such as timed voiding training, pelvic floor muscle training or education for patients and medication is commonly used. But anticholinergic drugs mainly used in medication have the possibility of the side effects such as dry mouth, constipation, voiding dysfunction, central nervous system symptoms or the like and the therapeutic efficacy is sometimes insufficient. Therefore, the early development of new drugs that have high safety and potency has been desired (see non-Patent Reference 4).

It has been reported that the compound (-)-1-(3-hydroxypropyl)-5-((2R)-2-{[2-({2-[(2,2,2-trifluoroethyl)-oxy]phenyl}oxy)ethyl]amino}propyl)-2,3-dihydro-1H-indol-7-carboxamide or a pharmaceutically acceptable salt thereof have the selective inhibitory effects against the urethral smooth muscle contraction and decrease the urethral pressure without a significant effect on the blood pressure, and they are extremely useful compounds as drugs for the treatment of the voiding dysfunction or the like induced by BPH (see Patent Reference 1). But it has been neither reported nor suggested that this compound is useful for the prevention or treatment of OAB accompanied with neurogenic disorders.

Patent Reference 1: Japanese Patent Publication H6-220015;
Non-patent Reference 1: Yukio Honma, et. Al., [The standardization of terminology of lower urinary tract function: report from the standardization sub-committee of the international continence society], Journal of Neurogenic bladder society, 2003, Vol.14, No.2, pp.278-289;
Non-patent Reference 2: Yukio Honma, et. al., [Epidemiologic survey on urination], Journal of Neurogenic bladder society, 2003, Vol.14, No.2, pp.266-277;
Non-patent Reference 3: Osamu Yamaguchi, Mechanism of overactive bladder, PHARMACIA SCOPE, published by Pharmacia Company, 2003, Vol.42, No.4, pp.12-13;
Non-patent Reference 4: Osamu Nishimura, What is overactive bladder (OAB)?, PHARMACIA SCOPE, published by Pharmacia Company, 2003, Vol.42, No.1, pp.14-15.
JP 2001-288115A discloses an alpha-1-receptor antagonist as a remedy for lower urinary tract symptoms.
JP 2000-247998A relates to the therapeutic agent for dysuria accompanying prostatic hypertrophy which contains the alpha 1A adrenoreceptor antagonist.

WO 99/15202 relates to remedies for dysuria resulting from prostatic hypertrophy which contain a highly selective alpha 11-adrenergic receptor blocker as the active ingredient and do not affect blood pressure.

WO 99/48530 relates to combination therapy for the treatment of benign prostatic hyperplasia comprising an alpha-1a antagonist and an endothelin antagonist.

US 2002/1432007 describes nitrosated and/or nitrosylated α-adrenergic receptor antagonists, compositions containing them and methods for treating or preventing benign prostatic hyperplasia, hypertension, congestive heart failure, variant angina, glaucoma, neurodegenerative disorders, vasospastic diseases, cognitive disorders, urge incontinence or overactive bladder.

Yamada S. et al. Life Science Vol.62, No.17-18, 1998 pp.1585-1589 describes the binding properties of novel alpha1-adrenoceptor antagonists in prostate and other tissues of rats.

Okura T. et al. Journal of Pharmacy and Pharmacology, Vol.54, No.7, 2002 pp.975-998 examines the ex-vivo occupancy by KMD-3213 of alphal-adreno receptors in the prostate and other tissues of rats in terms of tissue selectivity and duration of occupancy in relation to plasma concentration.

Akiyama K. et al., Japanese Journal of Pharmacology, Vol.90, No.2, 2002 pp.131-137 describes an investigation into the effects of KMD-3213 on the tilt-induced blood pressure response in anesthetized normotensive rats.

Okashi T. et al., 'Urination Disorders and Incontinence' Hekkan Hondanren, No.591, 1998, pp. 57-60 discusses administering an α_{1A} blocker as an antihypertensive agent for elderly patients with hypertension.

Yoshida M. et al., Folia Pharmacologica Japonica, Vol.121, No.5, 2003, pp.307-316 presents data on functional responses in physiological and pathological conditions and the interactions between neurotransmitters and neurons in lower urinary tract function.

Ishidura O. et al., Geriatric Medicine, Vol.41, No.8, 2003 pp.1149-1153 provides an overview of the relationship between overactive bladder and hypertrophy of the prostate gland.

Ishizuka O. et al., BR.J. Pharmacol., Vol.117, No.5, 1996, pp.962-966 examines the response to doxazosin in rats with and without bladder outlet obstruction.

WO 2004/054574 discloses a solid oral dosage form pharmaceutical for the treatment of dysuria comprising KMD-3213.

WO 2004/022538 discloses a crystal of KMD-3213 as a useful therapeutic agent for dysuria.

R.A. Appell, Reviews in Urology, Vol.5, No.8, 2003, pp.537-541 describes the treatment of overactive bladder in special patient populations including overactive bladder of neurogenic origin.

### Disclosure of the Invention

### Problem to be solved by the Invention

The object of the present invention is to provide pharmaceutical compositions useful for the prevention or treatment of OAB accompanied with neurogenic disorders.

### Means of solving the Problems

The present inventors have studied earnestly to find a compound useful for the prevention or treatment of OAB accompanied with neurogenic disorders, and found that (-)-1-(3-hydroxypropyl)-5-((2R)-2-{[2-({2-[(2,2,2-trifluoroethyl)-oxy]phenyl}oxy)ethyl]amino}propyl)-2,3-dihydro-1H-indol-7-carboxamide remarkably inhibits the frequency of involuntary contraction in filling phase and has an effect to prolong the micturition interval, thereby forming the basis of the present invention.

That is, present invention relates to:
[1] a pharmaceutical composition for use in the prevention or treatment of urinary urgency or frequency in overactive bladder accompanied with neurogenic disorders, which comprises as an active ingredient (-)-1-(3-hydroxypropyl)-5-((2R)-2-{[2-({2-[(2,2,2-trifluoroethyl)-oxy]phenyl}oxy)ethyl]amino}propyl)-2,3-dihydro-1H-indol-7-carboxamide or a pharmaceutically acceptable salt thereof;
[2] a pharmaceutical composition as described in the above [1] wherein the neurogenic disorder is cerebral infarction, Parkinson's disease, spinal cord involvement, peripheral neurogenic disorder or multiple sclerosis;
[3] a pharmaceutical composition as described in the above [1] or [2] which is used in combination with one or more other agents used for overactive bladder accompanied with neurogenic disorder;
[4] a pharmaceutical composition as described in the above [3] wherein the other agent used for overactive bladder accompanied with neurogenic disorder is an agent selected from an anticholinergic drug, an anti-anxiety drug, a cholinergic drug, a cholinesterase inhibitor, an antispasmodic drug, an anti-inflammatory drug and an antimicrobial drug; and the like.

In the present invention, the term "overactive bladder (OAB) accompanied with neurogenic disorder" means overactive bladder defined by the above new standardization of terminology of ICS, which is caused by a neurogenic disorder. As the neurogenic disorder, cerebrovascular disorders such as cerebral infarction, cerebral apoplexy and the like, Parkinson' s disease, spinal cord involvement such as spinal cord injury and the like, peripheral neurogenic disorders accompanied with diabetes or the like, multiple sclerosis and the like can be illustrated. The OAB accompanied with neurogenic disorders includes neurogenic bladder (for example, that caused by cerebrovascular disorders, spinal cord involvement, diabetic neuropathy, multiple sclerosis or the like) and unstable bladder, but does not include that caused by lower urinary tract obstructive diseases such as benign prostatic hypertrophy.

The present inventors confirmed that (-)-1-(3-hydroxypropyl)-5-((2R)-2-{[2-({2-[(2,2,2-trifluoroethyl)-oxy]phenyl}oxy)ethyllamino}propyl)-2,3-dihydro-1H-indol-7-carboxamide exerts an effect to inhibit the frequency of involuntary contraction in filling phase and an effect to prolong the micturition interval by pharmacological tests using rats with spinal cord injuries. These results support the usefulness of the present compounds for the urinary urgency and frequency in OAB accompanied with neurogenic disorder, and therefore, it has been shown that (-)-1-(3-hydroxypropyl)-5-((2R)-2-{[2-({2-[(2,2,2-trifluoroethyl)-oxy]phenyl}oxy)ethyl]amino}propyl)-2,3-dihydro-1H-indol-7-carboxamide is extremely useful for the prevention or treatment of OAB accompanies with neurogenic disorder.

In the present invention the compound (-)-1-(3-hydroxypropyl)-5-((2R)-2-{[2-({2-[(2,2,2-trifluoroethyl)oxy]phenyl}oxy)ethyl]amino}propyl)-2,3-dihydro-1H-indol-7-yl-carboxamide and pharmaceutically acceptable salts thereof (among them, a dihydrobromide is hereinafter referred to as "compound 1") can be illustrated

Several manufacturing methods of the compound of the present invention are known, and they can be easily prepared in a method described in literatures or the like (see the above Patent Reference 1).

As the pharmaceutically acceptable salt thereof, a salt with an inorganic base such as sodium, potassium, calcium or the like, a salt with an organic amine such as morpholine, piperidine or the like, a salt with a mineral acid such as hydrochloric acid, hydrobromic acid, sulfuric acid or the like, a salt with an organic acid such as methanesulfonic acid, benzenesulfonic acid, p-toluenesulfonic acid, acetic acid, citric acid, succinic acid, tartaric acid, 2,4-dimethyl-benzenesulfonic acid, 2,5-dimethylbenzenesulfonic acid, 2,4,6-trimethylbenzenesulfonic acid, (+)-camphorsulfonic acid, (-)-camphorsulfonic acid, 4-chlorobenzenesulfonic acid, 2-naphthalenesulfonic acid, 1-butanesulfonic acid, fumaric acid, glutamic acid, aspartic acid and the like can be illustrated.

The compound of the present invention includes its hydrates and solvates with pharmaceutically acceptable solvents such as ethanol or the like. In addition, the compound of the present invention includes both of amorphous forms and crystal forms, and a single polymorph, a mixture of two or more polymorphs and a mixture of a polymorph and an amorphous form thereof.

Furthermore, the compound of the present invention can be also used in combination with one or more other drugs used for OAB accompanied with neurogenic disorder. Examples of the other drugs which can be used in combination with include, for example, an anticholinergic drug (tolterodine, oxybutynin, propiverine or the like), an anti-anxiety drug, a cholinergic drug (bethanechol chloride or the like), a cholinesterase inhibitor (distigmine bromide or the like), an antispasmodic drug (flavoxate or the like), an anti-inflammatory drug and an antimicrobial drug and the like.

In the case of uses of the compound of the present invention in combination with the above one or more other drugs, the present invention includes either dosage forms of simultaneous administration as a single preparation or separated preparations in way of the same or different administration route, and administration at different dosage intervals as separated preparations in way of the same or different administration route. A pharmaceutical combination comprising the compound of the present invention and the above other drug(s) includes both dosage forms as a single preparation and separated preparations for combination as mentioned above.

The compound of the present invention can obtain more advantageous effects than additive effects in the prevention or treatment of the above diseases when using suitably in combination with the above one or more other drugs. Also, the administration dose can be decreased in comparison with administration of either drug alone, or adverse effects of the above drugs coadministered can be avoided or declined.

When the pharmaceutical compositions of the present invention are employed in the practical treatment, various dosage forms are used depending on their uses. As examples of the dosage forms, powders, granules, fine granules, dry syrups, tablets, capsules, injections, solutions, ointments, suppositories, poultices and the like are illustrated, which are orally or parenterally administered.

These pharmaceutical compositions can be prepared by suitably admixing with or by diluting and dissolving with an appropriate pharmaceutical additive such as excipients, disintegrators, binders, lubricants, diluents, buffers, isotonicities, antiseptics, moistening agents, emulsifiers, dispersing agents, stabilizing agents, dissolving aids and the like, and formulating the mixture in accordance with conventional methods. In the case of the uses in combination with other drug(s), they can be prepared by formulating each active ingredient together or individually in a similar manner as defined above.

For example, powders can be formulated by, if desired, admixing well a compound of the present invention with appropriate excipients, lubricants and the like.

For example, tablets can be formulated by, if desired, admixing a compound of the present invention with appropriate excipients, disintegrating agents, binders, lubricants and the like, and compressing the mixture in accordance with conventional methods. The tablets, further if desired, can be suitably coated to provide film-coated tablets, sugar-coated tablets, enteric-coated tablets and the like.

For example, capsules can be formulated by, if desired, admixing well a compound of the present invention with appropriate excipients, lubricants and the like, or formulating granules or fine-powders in accordance with conventional methods, and then filling the compositions in appropriate capsules.

When the pharmaceutical compositions of the present invention are employed in the practical treatment, the dosage of a compound represented by the above general formula as the active ingredient is appropriately decided depending on the body weight, age, sex and degree of diseases or treatment of each patient, which is approximately within the range of from 0.5 to 500 mg per day per adult human in the case of oral administration and approximately within the range of from 0.05 to 100 mg per day per adult human in the case of parenteral administration, and the daily dose can be divided into one to several doses per day and administered. Also, in the case of the uses in combination with the above other drug(s), the dosage of the compound of the present invention can be decreased depending on the dosage of the other drug(s).

### Effect of the Invention

The pharmaceutical compositions of the present invention exert an excellent improving effect on frequency of involuntary contraction and micturition interval as indicators of urinary urgency and frequency in OAB accompanied with neurogenic disorder such as spinal cord injury or the like. The present invention can provide a pharmaceutical composition useful for the prevention or treatment of OAB accompanied with neurogenic disorder.

### Brief description of Drawings

[Figure 1]

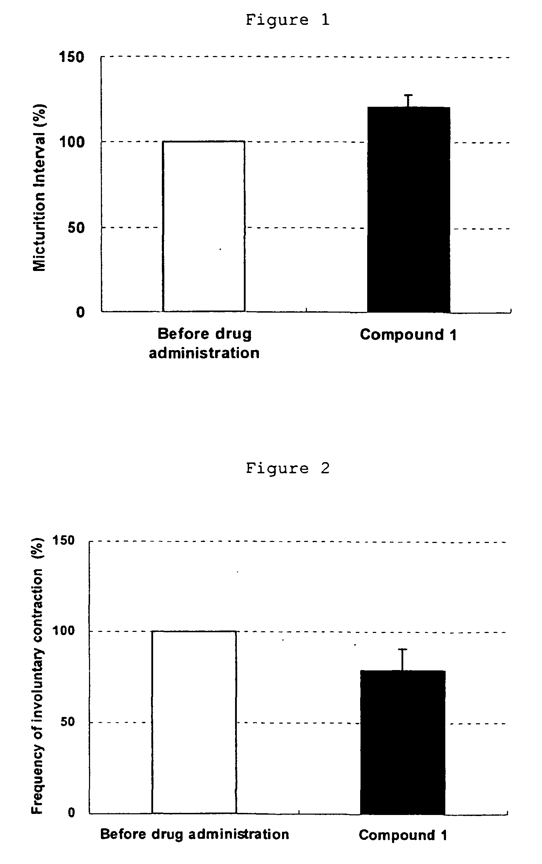

Figure 1 shows the effects on the micturition interval in rat spinal cord injured OAB model. The "□" shows data before drug administration and the "■" shows data on compound 1. The vertical axis indicates the percentage of the micturition interval against the value before drug administration.

[Figure 2]
Figure 2 shows the effects of the compound on the frequency of involuntary contraction in rat spinal cord injured OAB model. The "□" shows data before drug administration and the "■" shows data on compound 1. The vertical axis indicates the percentage of the frequency of involuntary contraction against the value before drug administration.

### Best Mode to practice the Invention

The present invention is further illustrated in more detail by way of the following Example.

### Example 1

### Efficacy on urodynamic study in rat spinal cord injured OAB model

In ether anesthetized female rats, spinal cord transaction was performed at the level of Th10. About 1 month after the spinal cord transaction, each rat was anesthetized with pentobarbital and a catheter filled with saline was implanted into the urinary bladder, ligated, secured on the back of the neck and closed. Seven days after the bladder catheter implantation, another catheter filled with heparin-containing saline was implanted into the carotid vein, and ligated, secured on the back of the neck and closed. The next day cystometry was performed in the conscious rate under free moving. Saline was instilled into the urinary bladder at a rate of 12 mL/hr. A drug was injected through the carotid vein catheter that was secured on the back of the neck. As a result, in this female rat spinal cord injured model, involuntary contractions were observed in filling phase. Intravenous injection of compound 1 (0.1 mg/kg) prolonged the micturition interval by about 20% (Figure 1) and decreased the frequency of involuntary contraction in filling phase by 20% (Figure 2) in the same model.

As mentioned above, it was shown that the compound of the present invention exerts an excellent improving effect on the frequency of the involuntary contraction and micturition interval at the parameter of urgency and frequency, respectively, in spinal cord injured OAB model, extremely useful for the prevention or treatment of OAB associated with neurogenic disorders.

### Industrial Applicability

The pharmaceutical compositions of the present invention exert an excellent improving effects on urinary urgency and frequency and are extremely useful as an agent for the prevention or treatment of OAB accompanied with neurogenic disorders.

## Claims

1. A pharmaceutical composition for use in the prevention or treatment of urinary urgency or frequency in overactive bladder accompanied with neurogenic disorders, which comprises as an active ingredient (-)-1-(3-hydroxypropyl)-5-((2R)-2-{[2-({2-[(2,2,2-trifluoroethyl)oxy]phenyl}oxy)-ethyl]amino}propyl)-2,3-dihydro-1H-indol-7-carboxamide or a pharmaceutically acceptable salt thereof.

2. A pharmaceutical composition as claimed in claim 1 wherein the neurogenic disorder is cerebral infarction, Parkinson's disease, spinal cord involvement, peripheral neurogenic disorder or multiple sclerosis.

3. A pharmaceutical composition is claimed in claim 1 or 2, which is used in combination with one of more other agents used for overactive bladder accompanied with neurogenic disorder.

4. A pharmaceutical composition as claimed in claim 3 wherein the other agent used for overactive bladder accompanied with neurogenic disorder is an agent selected from an anticholinergic drug, an anti-anxiety drug, a cholinergic drug, a cholinesterase inhibitor, an antispasmodic drug, an anti-inflammatory drug and an antimicrobial drug.

## Patentansprüche

1. Pharmazeutische Zusammensetzung zur Verwendung bei der Vorbeugung oder Behandlung von Harndrang oder ständiger Blasenentleerung bei überaktiver Blase begleitet von neurogenen Störungen, die als Wirkstoff (-)-1-(3-Hydroxypropyl)-5-((2R)-2-{[2-({2-[(2,2,2-trifluorethyl)oxy]phenyl}oxy)-ethyl]amino}propyl)-2,3-dihydro-1H-indol-7-carboxamid oder ein pharmazeutisch akzeptables Salz davon enthält.

2. Pharmazeutische Zusammensetzung nach Anspruch 1, worin es sich bei der neurogenen Störung um Hirninfarkt, Parkinson-Krankheit, Rückenmarksbeteiligung, periphere neurogene Störung oder Multiple Sklerose handelt.

3. Pharmazeutische Zusammensetzung nach Anspruch 1 oder 2, die in Kombination mit einem oder mehreren anderen Wirkstoffen für eine überaktive Blase begleitet von einer neurogenen Störung verwendet wird.

4. Pharmazeutische Zusammensetzung nach Anspruch 3, worin der andere Wirkstoff, der für eine überaktive Blase begleitet von einer neurogenen Störung verwendet wird, ein Wirkstoff ist, der unter einem Anticholinergikum, einem Anxiolytikum, einem cholinergen Wirkstoff, einem Cholinesterase-Hemmer, einem krampflösenden Wirkstoff, einem entzündungshemmenden Wirkstoff und einem antimikrobiellen Wirkstoff ausgewählt ist.

## Revendications

1. Composition pharmaceutique pour utilisation dans la prévention ou le traitement de la miction impérieuse ou de la fréquence urinaire exagérée dans l'hyperactivité vésicale accompagnée de troubles neurogènes, qui comprend comme ingrédient actif du (-)-1-(3-hydroxypropyl)-5-((2R)-2-{[2-({2-[(2,2,2-trifluoréthyl)oxy]phényl}oxy)éthyl]amino)-propyl)-2,3-dihydro-1H-indole-7-carboxamide ou un de ses sels pharmaceutiquement acceptables.

2. Composition pharmaceutique suivant la revendication 1, dans laquelle le trouble neurogène est un infarctus cérébral, la maladie de Parkinson, une atteinte de la moelle épinière, un trouble neurogène périphérique ou la sclérose en plaques.

3. Composition pharmaceutique suivant la revendication 1 ou 2, qui est utilisée en association avec un ou plusieurs autres agents utilisés pour l'hyperactivité vésicale accompagnée d'un trouble neurogène.

4. Composition pharmaceutique suivant la revendication 3, dans laquelle l'autre agent utilisé pour l'hyperactivité vésicale accompagnée d'un trouble neurogène est un agent choisi entre un médicament anti-cholinergique, un anxiolytique, un médicament cholinergique, un inhibiteur de cholinestérase, un médicament anti-spasmodique, un médicament anti-inflammatoire et un médicament anti-microbien.
